(19) Europäisches Patentamt
European Patent Office
Office européen des brevets

(11) Veröffentlichungsnummer: **0 084 893**
**B1**

# (12) EUROPÄISCHE PATENTSCHRIFT

(45) Veröffentlichungstag der Patentschrift: **14.01.87**

(51) Int. Cl.⁴: **C 07 D 265/22**, A 01 N 43/86

(21) Anmeldenummer: **83100793.5**

(22) Anmeldetag: **11.04.80**

## (54) 4H-3,1-Benzoxazinderivate, Verfahren zu ihrer Herstellung und ihre Verwendung zur Bekämpfung unerwünschten Pflanzenwuchses.

(30) Priorität: **12.04.79 DE 2914915**

(43) Veröffentlichungstag der Anmeldung: **03.08.83 Patentblatt 83/31**

(45) Bekanntmachung des Hinweises auf die Patenterteilung: **14.01.87 Patentblatt 87/3**

(84) Benannte Vertragsstaaten: **AT BE CH DE FR GB IT LI LU NL SE**

(56) Entgegenhaltungen:
**BE-A-648 259**
**DE-A-2 241 012**
**DE-A-2 556 590**
**US-A-3 914 121**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Hamprecht, Gerhard, Dr., Rote- Turm-Strasse 28, D-6940 Weinheim (DE)**
Erfinder: **Wuerzer, Bruno, Dr., Dipl.- Landwirt, Ruedigerstrasse 13, D-6701 Otterstadt (DE)**

EP 0 084 893 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung ist eine Ausscheidung aus der Patentanmeldung Nr. 80101957.1 (EP-A-17 931) und betrifft 4H-3,1-Benzoxazinderivate, ein Verfahren zu ihrer Herstellung, Herbizide, die diese Verbindungen als Wirkstoffe enthalten, und Verfahren zur Bekämpfung unerwünschten Pflanzenwuchses mit diesen Verbindungen.

Substituierte 4H-3,1-Benzoxazin-4-one sind bekannt als Zwischenprodukte für die Synthese pharmakologisch wirksamer Verbindungen (DE-A- 1 670 375, DE-A- 2 556 590) oder als herbizide Wirkstoffe, wobei insbesondere 4H-3,1-Benzoxazin-4-one, die in 2-Stellung einen gegebenenfalls substituierten Phenylrest tragen, herbizid wirksam sind (BE-A- 648 259, US-A- 3 970 652, US-A- 3 914 121). Die bekannten Verbindungen zeichnen sich durch eine gute Verträglichkeit bei einer Reihe von Kulturen, wie Getreidearten, Reis, Mais und Kultursorghum, aus. Ihre Schwäche liegt in einem eng begrenzten Wirkungsspektrum gegen breitblättrige unerwünschte Pflanzen. Darüber hinaus sind selbst bei der Bekämpfung von Pflanzen, gegen die diese Benzoxazine wirksam sind, relativ hohe Aufwandmengen pro Flächeneinheit erforderlich.

Es wurde nun gefunden, daß 4H-3,1-Benzoxazinderivate der Formel (I)

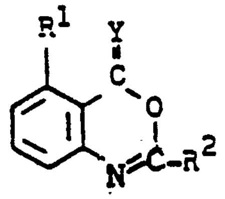

I

in der

Y Sauerstoff oder Schwefel,

$R^1$ Fluor, Chlor oder Brom und

$R^2$ einen durch Chlormethoxy, Fluormethoxy, Difluormethoxy, Difluorchlormethoxy, Trifluormethoxy, Trichlormethoxy, 1,1,2,2-Tetrafluoräthoxy, 1,1,2-Trifluor-2-chloräthoxy, 1,1,1-Trifluor-2-bromäthoxy, 1,1,2,3,3,3-Hexafluor-n-propyloxy, Pentafluoräthoxy, Hexafluorisopropoxy, Difluormethylmercapto, Trifluormethylmercapto, Pentafluoräthylmercapto 1,1,2,2-Tetrafluoräthylmercapto, Trichlormethylmercapto oder Dichlorfluormethylmercapto in m- oder p-Stellung substituierten Phenylrest, der auch durch Difluorchlormethylmercapto substituiert sein kann, wenn $R^1$ für Chlor und Y für O steht, oder der auch unsubstituiert sein kann, wenn $R^1$ für Fluor oder Chlor steht,

bedeuten, neben vorzüglicher Kulturpflanzenverträglichkeit eine wesentlich stärkere herbizide Wirkung zeigen als bekannte herbizide Mittel, die Benzoxazine als Wirkstoffe enthalten.

Man erhält Benzoxazinderivate der Formel (I) dadurch, daß man eine gegebenenfalls substituierte Anthranilsäure der Formel (II)

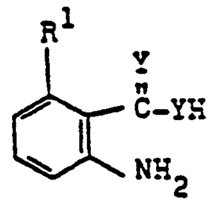

II

in der

$R^1$ und Y die oben genannten Bedeutungen haben, mit mindestens dem zweifachen molaren Überschuß eines Carbonsäurehalogenids der Formel (III)

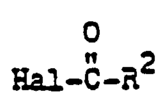

III

in der

$R^2$ die oben genannten Bedeutungen hat und

Hal für Halogen, insbesondere Fluor, Chlor oder Brom, steht,

in einem aromatischen tertiären Amin als Lösungsmittel bei einer Temperatur im Bereich zwischen 10 und 60°C umsetzt.

Zweckmäßigerweise läßt man einen zweifachen Überschuß des Carbonsäurehalogenids der Formel (III) in eine Lösung der gegebenenfalls substituierten Anthranilsäure der Formel (II) in der 5- bis 25-fachen molaren

Menge eines aromatischen Amins, bezogen auf Anthranilsäure, bei einer Temperatur zwischen 10 und 60°C einlaufen und rührt dann 30 Minuten bei 25°C nach (s. J. Chem. Soc. (C) 1968, 1593). Zur Aufarbeitung kann dann Eiswasser eingerührt und vom ausgefallenen Niederschlag abgesaugt werden. Ebenso können auch das Carbonsäurehalogenid vorgelegt und die Anthranilsäure der Formel (II) zugegeben werden.

Geeignete aromatische tertiäre Amine sind beispielsweise Pyridin, α-,β-,γ-Picolin, Lutidin, Chinolin und Acridin.

Die Benzoxazinderivate der Formel (I) können auch dadurch erhalten werden, daß man eine gegebenenfalls substituierte Anthranilsäure der Formel (II)

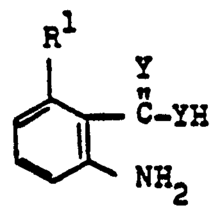

II

in der
$R^1$ und Y die oben genannten Bedeutungen haben, oder ein Alkalimetall- oder Erdalkalimetallsalz dieser Anthranilsäure mit ungefähr stöchiometrischen Mengen eines Carbonsäurehalogenids der Formel (III)

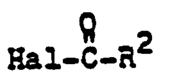

III

in der
$R^2$ die im Anspruch 1 genannten Bedeutungen hat und
Hal für Halogen steht,
in einem inerten organischen Lösungsmittel oder in Wasser und gegebenenfalls in Anwesenheit eines Säureacceptors bei einer Temperatur im Bereich von 0 bis 60°C zu einem Carbonamid der Formel (IV)

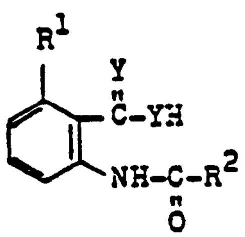

IV

in der
$R^1$, $R^2$ und Y die obengenannten Bedeutungen haben, umsetzt und dieses dann in Gegenwart eines wasserentziehenden Mittels bei einer Temperatur zwischen 30 und 150°C cyclisiert.

Geeignete inerte Lösungsmittel sind Kohlenwasserstoffe, wie Ligroin, Benzin, Toluol, Pentan, Hexan, Cyclohexan, Petroläther, Halogenkohlenwasserstoffe, wie Methylenchlorid, Chloroform, Tetrachlorkohlenstoff, 1,1- und 1,2-Dichloräthan, 1,1,1- und 1,1,2-Trichloräthan, Chlorbenzol, o-, m-, p-Dichlorbenzol, o-, m-, p-Chlortoluol, Nitrokohlenwasserstoffe, wie Nitrobenzol, Nitroäthan, o-, m-, p-Chlornitrobenzol, Nitrile, wie Acetonitril, Butyronitril, Isobutyronitril, Äther, wie Diäthyläther, Di-n-propyläther, Tetrahydrofuran, Dioxan, Ester, wie Acetessigester, Äthylacetat oder Isobutylacetat, oder Amide, wie Formamid, Methylformamid oder Dimethylformamid.

Als Säureacceptoren können alle üblichen Säurebindemittel verwendet werden. Hierzu gehören vorzugsweise Alkalihydroxide, Alkalicarbonate und tertiäre organische Basen. Als besonders geeignet seien im einzelnen genannt: Natriumhydroxid, Natriumcarbonat, Natriumhydrogencarbonat, Triäthylamin, Pyridin, Trimethylamin, α-,β-,γ-Picolin, Lutidin, N,N-Dimethylanilin, N,N-Dimethylcyclohexylamin, Chinolin, Tri-n-propylamin und Tri-n-butylamin. Das Säurebindemittel wird zweckmäßigerweise in äquivalenten Mengen, bezogen auf Carbonsäurehalogenid der Formel (III), eingesetzt.

Als wasserentziehende Mittel können symmetrische und gemischte Carbonsäureanhydride, wie Essigsäureanhydrid, Propionsäureanhydrid, Buttersäureanhydrid, Ameisensäureessigsäureanhydrid, Ameisensäurepropionsäureanhydrid oder Essigsäurepropionsäureanhydrid, ferner Dicyclohexylcarbodiimid oder Thionylchlorid verwendet werden. Die Cyclisierung wird unter Zusatz der 1- bis 10-fachen molaren Menge an wasserentziehendem Mittel, bezogen auf Carbonamid der Formel (IV), durchgeführt.

Die Ausgangsstoffe der Formeln (II) und (III) werden in ungefähr stöchiometrischem Verhältnis eingesetzt, d.h. in einen Unter- bzw. Überschuß von bis zu 10 % Ausgangsstoff der Formel (III) gegenüber Ausgangsstoff

der Formel (II).

Zweckmäßigerweise wird das Verfahren so durchgeführt, daß man über zwei Zuführungen das Carbonsäurehalogenid der Formel (III) und die äquivalente Menge an Säureacceptor bei einer Temperatur zwischen 0 und 60°C zu einer ungefähr äquivalenten Menge der Anthranilsäure der Formel (II) bzw. ihres Salzes in einem inerten organischen Lösungsmittel bzw. in Wasser zulaufen läßt. Dann rührt man 15 Minuten bei Raumtemperatur nach. Das Reaktionsgemisch wird dann gegebenenfalls eingeengt, in der Wärme mit 5 n Salzsäure angesäuert, abgekühlt und abgesaugt (J. Org. Chem. 2, 396 (1944)), wobei eine N-Acyl-2-aminobenzoesäure erhalten wird. Diese kann man in Gegenwart der 5- bis 10-fachen Menge Acetanhydrid durch Rühren unter Rückfluß - gegebenenfalls unter Abdestillation der entstandenen Essigsäure - zu dem gewünschten 4H-3,1-Benzoxazin cyclisieren. Zur Aufarbeitung entfernt man hierbei überschüssiges Acetanhydrid am Rotationsverdampfer unter vermindertem Druck und kristallisiert gegebenenfalls zur Reinigung um. Anstelle der Anthranilsäure kann auch das Carbonsäurehalogenid vorgelegt werden.

Anstelle von Acetanhydrid kann man jedoch auch mit der 1- bis 4-fachen Menge Dicyclohexylcarbodiimid oder Thionylchlorid bei 30 bis 150°C cyclisieren.

Im Falle fluoralkoxy- bzw. fluoralkylmercapto-substituierter 2-Phenyl-3,1-benzoxazin-4-one überführt man zweckmäßigerweise eine fluoralkoxy- bzw. fluoralkylmercapto-substituierte Benzoesäure nach üblichen Methoden in die entsprechenden Säurechloride (Houben-Weyl, Methoden der organischen Chemie, Bd. 8, S. 463 ff, 4. Auflage, Georg Thieme-Verlag, Stuttgart 1952), die dann mit gegebenenfalls substituierten Anthranilsäuren auf bekannte Weise in die entsprechenden Amide umgewandelt werden. Letztere werden dann durch Ringschluß in Gegenwart wasserabspaltender Agentien in substituierte 2-Phenyl-3,1-benzoxazin-4-one überführt.

Zur Isolierung der 4H-3,1-Benzoxazinderivate der Fomel (I) aus der Reaktionsmischung kann man diese mit Wasser, verdünntem Alkali oder verdünnter Säure zur Abtrennung von Nebenprodukten, wie nicht umgesetzter Anthranilsäure, Säurechlorid bzw. Basenhydrochlorid, behandeln, trocknen und dann einengen. Gegebenenfalls können die Endprodukte auch durch Umkristallisation oder Chromatographie gereinigt werden.

Als Vorprodukte für 4H-3,1-Benzoxazin-4-one lassen sich folgende Carbonsäurehalogenide der Formel (III) herstellen:

3-(1',1',2'-Trifluor-2'-chloräthoxy)-benzoylchlorid

52,4 Gewichtsteile Chlortrifluoräthylen werden während 10 Stunden bei 45 bis 52°C unter Rückfluß in eine Mischung von 46,5 Gewichtsteilen 3-Hydroxybenzoesäuremethylester und 9,5 Gewichtsteilen Kaliumhydroxidpulver in 50 Gewichtsteilen Aceton eingeleitet. Nach dem Einengen am Rotationsverdampfer unter vermindertem Druck wird das Reaktionsgemisch in Methylenchlorid aufgenommen, mit Natriumhydrogencarbonatlösung extrahiert, getrocknet und eingeengt, wobei 69,5 Gewichtsteile 3-(1',1',2'-Trifluor-2'-chloräthoxy)-benzoesäuremethylester vom $n_D^{25}$ = 1.4710 erhalten werden.

40 Gewichtsteile 3-(1',1',2'-Trifluor-2'-chloräthoxy)-benzoesäuremethylester werden in einer Mischung von 8,4 Gewichtsteilen Kaliumhydroxid in 100 Gewichtsteilen Wasser und 5 Gewichtsteilen Tetrahydrofuran 15 Minuten bei 95°C gerührt. Die entstandene Lösung wird mit konz. Salzsäure angesäuert, der ausgefallene Niederschlag wird abgesaugt und getrocknet; dabei werden 35 Gewichtsteile 3-(1',1',2'-Trifluor-2'-chloräthoxy)-benzoesäure vom Fp. 79 bis 85°C isoliert.

35 Gewichtsteile 3-(1',1',2'-Trifluor-2'-chloräthoxy)-benzoesäure werden mittels 20,2 Gewichtsteilen Thionylchlorid und 0,2 Gewichtsteilen Pyridin als Katalysator in üblicher Weise in das 3-(1',1',2'-Trifluor-2'-chloräthoxy)-benzoylchlorid vom $n_D^{22}$ = 1.4900 (IR: C=O 1760, 1742 $cm^{-1}$) übergeführt; Ausbeute: 34,5 Gewichtsteile, entsprechend 92 % der Theorie.

Im folgenden wird die Herstellung von 4H-3,1-Benzoxazinderivaten beispielhaft erläutert. Gewichtsteile verhalten sich zu Volumenteilen wie Kilogramm zu Liter.

1. Herstellung von 2-(m-1',1',2',2'-Tetrafluoräthoxy-phenyl)-3,1-benzoxazin-4-on

39,4 Gewichtsteile Thionylchlorid werden zu einer Suspension von 65 Gewichtsteilen m-(1,1,2,2-Tetrafluoräthoxy)-benzoesäure in 500 Volumenteilen 1,2-Dichloräthan gegeben und dann 3 Stunden lang unter Rühren unter Rückfluß erhitzt. Nach den Einengen unter vermindertem Druck und dem Absaugen von wenig wieder ausgefallenem Ausgangsmaterial wird das m-(1,1,2,2-Tetrafluoräthoxy)-benzoylchlorid als gelbliches Öl erhalten. Das IR-Spektrum zeigt Banden für C=O bei 1770, 1748 $cm^{-1}$, für Fluoralkoxy bei 1225, 1190, 1125 $cm^{-1}$.

25,7 Gewichtsteile m-(1,1,2,2-Tetrafluoräthoxy)-benzoylchlorid und 10,1 Gewichtsteile Triäthylamin werden unter Rühren über zwei Zuführungen innerhalb von 15 Minuten zu einer Mischung von 13,7 Gewichtsteilen Anthranilsäure in 300 Volumenteilen 1,2-Dichloräthan gegeben und 12 Stunden lang bei Raumtemperatur gerührt. Das Reaktionsgemisch wird mit 0,5 n Salzsäure und mit Wasser extrahiert, über Magnesiumsulfat getrocknet und dann unter vermindertem Druck eingeengt. Nach dem Anreiben in 0,5 n Salzsäure, Absaugen und Waschen mit Wasser wird m-(1,1,2,2-Tetrafluoräthoxy)-benzoylanthranilsäure vom Fp. 159-163°C erhalten.

21 Gewichtsteile des so erhaltenen Produkts werden unter Rühren während 3 Stunden mit 200 Volumenteilen Acetanhydrid unter Rückfluß cyclisiert. Das Reaktionsgemisch wird unter vermindertem Druck eingeengt, in Methylenchlorid aufgenommen und über neutrales Aluminiumoxid chromatographiert. Nach dem Einengen werden 16 Gewichtsteile 2-(m-1',1',2',2'-Tetrafluoräthoxyphenyl)-3,1-benzoxazin-4-on vom Fp. 95-98°C erhalten.

2. Herstellung von 2-(m-Difluormethoxy-phenyl)-3,1-benzoxazin-4-on

260 Gewichtsteile Chlordifluormethan werden innerhalb von 1,5 Stunden unter Rühren bei 67-70°C in eine Mischung von 221 Gewichtsteilen m-Kresol, 412 Gewichtsteilen Natriumhydroxid, 600 Volumenteilen 1.4-Dioxan und 500 Volumenteilen Wasser eingeleitet. Nach 45 Minuten Rühren bei 68°C wird das Reaktionsgemisch abgekühlt, mit 1000 Volumenteilen Wasser verdünnt und viermal mit 200 Volumenteilen Äther extrahiert. Nach dem Trocknen, Einengen unter vermindertem Druck und Destillieren werden 172 Gewichtsteile m-Tolyldifluormethyläther vom Sdp. 64-67°C/24,7 mbar erhalten.

Eine Mischung von 47,4 Gewichtsteilen m-Tolyldifluormethyläther, 77 Gewichtsteilen Magnesiumsulfat, 134,3 Gewichtsteilen Kaliumpermanganat und 1900 Volumenteilen Wasser werden 3 Stunden lang bei 50-60°C und 2 Stunden lang bei 90°C gerührt. Nach dem Vernichten überschüssigen Permanganats mit Äthanol wird die Lösung noch heiß abgesaugt und dann das Filtrat angesäuert. Der ausgefallene Niederschlag wird in Methylenchlorid aufgenommen und getrocknet, wobei nach dem Einengen unter vermindertem Druck die 3-Difluormethoxybenzoesäure vom Fp. 85-87°C erhalten wird.

Die so erhaltene 3-Difluormethoxy-benzoesäure läßt sich mittels Thionylchlorid auf übliche Weise in das 3-Difluormethoxybenzoylchlorid vom $n_D^{25} = 1.5083$ überführen.

25 Gewichtsteile 3-Difluormethoxybenzoylchlorid und 12,2 Gewichtsteile Triäthylamin werden über 2 Zuführungen unter Rühren bei 25 bis 30°C zu einer Mischung von 16,6 Gewichtsteilen Anthranilsäure in 360 Gewichtsteilen 1,2-Dichloräthan innerhalb 15 Minuten gegeben. Nach 2 Stunden Rühren bei 25°C wird das Reaktionsgemisch mit 0,5 n Salzsäure und mit Wasser extrahiert. Anschließend wird die organische Phase viermal mit je 100 Teilen 0,5 n Natronlauge extrahiert und letztere in verd. Salzsäure eingerührt. Nach dem Absaugen und Trocknen werden 30,4 Gewichtsteile entsprechend 82 % d.Th., N-(3-Difluormethoxybenzoyl)-anthranilsäure vom Fp. 186 bis 191°C erhalten.

8,33 Gewichtsteile Thionylchlorid werden unter Rühren bei 25°C in eine Mischung von 18 Gewichtsteilen N-(3-Difluormethoxybenzoyl)-anthranilsäure in 250 Gewichtsteilen 1,2-Dichloräthan eingeführt; dann wird 4 Stunden unter Rückfluß gerührt. Nach dem Abkühlen wird das Reaktionsgemisch mit 100 Volumenteilen Eiswasser und 100 Volumenteilen 0,5 n Natronlauge extrahiert, getrocknet und über neutralem Aluminiumoxid chromatographiert. Dabei werden 12 Gewichtsteile, entsprechend 71 % der Theorie, 2-(m-Difluormethoxy-phenyl)-3,1-benzoxazin-4-on vom Fp. 84-87°C erhalten.

Nach entsprechenden Verfahren können folgende 4H-3,1-Benzoxazinderivate der Formel (I) hergestellt werden:

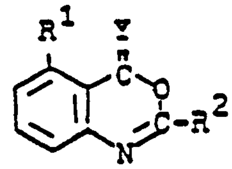

| $R^1$ | Y | $R^2$ | Fp. [°C] |
|---|---|---|---|
| Cl | O | $C_6H_5$ | 153-155 |
| Cl | S | $C_6H_5$ | |
| F | O | $C_6H_5$ | 157-161 |
| Cl | O | 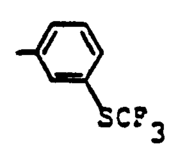 | 124-128 |
| Cl | O | 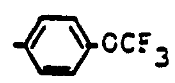 | 137-138 |
| F | O | 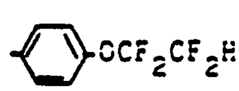 | 98-100 |
| F | O | 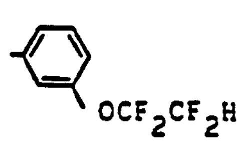 | 92- 96 |

| $R^1$ | Y | $R^2$ | Fp. [°C] |
|---|---|---|---|
| Cl | S | 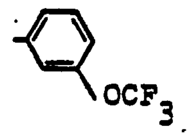 | |
| Cl | O | 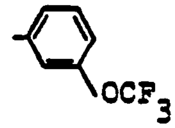 | 140-143 |
| F | O | 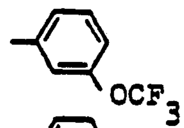 | 93-97 |
| Cl | O | 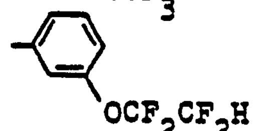 | 125-129 |
| F | O | 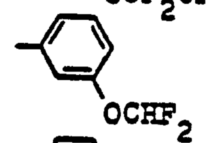 | 102-104 |
| Cl | O | 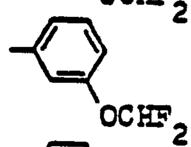 | 112-116 |
| Cl | O | 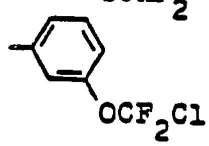 | 115-116 |
| Cl | O | 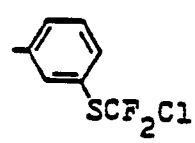 | |

Die Wirkstoffe können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen vcn mittlerem bis hohem Siedepunkt wie Kerosin oder Dieselöl, ferner Kohlenteeröle, sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate z.B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octyl-phenoläther, äthoxyliertes Isooctylphenol-, Octylphenol-, Nonylphenol,

Alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die erfindungsgemäßen Mittel enthalten als Formulierungen zwischen 0,1 und 95 Gewichtsprozent, vorzugsweise zwischen 0,5 und 90 Gewichtsprozent, Wirkstoff.

Der Einfluß der erfindungsgemäßen Mittel auf das Wachstum von unerwünschten Pflanzen wird durch Gewächshausversuche gezeigt:

Als Kulturgefäße dienten Plastikblumentöpfe mit 300 $cm^3$ Inhalt und lehmigem Sand mit etwa 1,5 % Humus als Substrat. Die Samen der Testpflanzen entsprechend Tabelle 1 wurden nach Arten getrennt flach eingesät oder es wurden transplantierte vorgekeimte Jungpflanzen oder Stecklinge transplantiert. Generell wurden sie zum Zwecke der Nachauflaufbehandlung bis zu einer Höhe von 3 bis 10 cm gezogen und danach behandelt. Die Wirkstoffe wurden auf die Sproßteile der Pflanzen und die nicht völlig von Pflanzen abgedeckte Bodenoberfläche, in Wasser als Verteilungsmittel suspendiert oder emulgiert, mittels fein verteilender Düsen gespritzt. Die Versuchstöpfe wurden dann in verschiedenen Temperaturbereichen der Gewächshausanlagen aufgestellt, wobei für wärmeliebende Arten 20 bis 30°C und für solche gemäßigter Klimate 10 bis 20°C bevorzugt wurden. Die Versuchsperiode erstreckte sich über 2 bis 4 Wochen. Während dieser Zeit wurden die Pflanzen gepflegt, und ihre Reaktion auf die einzelnen Wirkstoffe wurde ausgewertet. Die Auswertung der Versuche wurde nach einer Skala von 0 bis 100 vorgenommen. Dabei bedeutet 0 keine Schädigung und 100 völliges Absterben zumindest der oberirdischen Sproßteile.

Die in den Versuchen getesteten Pflanzenarten sind in Tabelle 1 aufgeführt.

Die Versuchsergebnisse in den Tabellen zeigen, daß Herbizide, die 4H-3,1-Benzoxazinderivate der Formel (I) enthalten, sich in Vergleich zu herbiziden Mitteln, die bekannte herbizide Benzoxazine enthalten, durch bessere herbizide Wirkung mit guter Selektivität bei einer Reihe von Kulturpflanzen auszeichnen. Der Schwerpunkt der Anwendung liegt in der Nachauflaufbehandlung der unerwünschten Pflanzen, sowohl auf Kulturflächen als auch auf unbebautem Land.

Sind die Kulturpflanzen gegenüber den Wirkstoffen weniger tolerant, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post directed, lay-by).

**Tabelle 1:** Liste der Pflanzennamen

| Botanischer Name | Abkürzung in Tabellen | Deutscher Name | Englischer Name |
|---|---|---|---|
| Arachis hypogaea | | Erdnuß | peanuts (groundnuts) |
| Avena sativa | | Hafer | oats |
| Beta vulgaris | Beta vulg. | Zuckerrübe | sugarbeets |
| Centaurea spp. | | Flockenblumenarten | knapweed |
| Chenopodium album | Chenopod. album | Weißer Gänsefuß | lambsquarters (goosefoot) |
| Chrysanthemum segetum | Chysanth. segetum | Saatwucherblume | corn marigold |
| Desmodium tortuosum | Desmod. tort. | | Florida beggarweed |
| Euphorbia geniculata | Euphorb. genic | südamerik. Wolfsmilchart | wild poinsettia |
| Glycine max | | Sojabohnen | soybeans |
| Hordeum vulgare | | Gerste | barley |
| Matricaria spp. | Matric. spp. | Kamillearten | chamomille |
| Malva neglecta | | | common mallow |

**Tabelle 1:** Liste der Pflanzennamen (Fortsetzung)

| Botanischer Name | Abkürzung in Tabellen | Deutscher Name | Englischer Name |
|---|---|---|---|
| Mercurialis annua | Mercurial. annua | Einjähriges Bingelkraut | annual mercury |
| Oryza sativa | | Reis | rice |
| Sesbania exaltata | | Turibaum | hemp sesbania (coffee-weed) |
| Solanum nigrum | Solan. nigr. | Schwarzer Nachtschatten | black nightshade |
| Sorghum bicolor | | Mohrenhirse (Kulturhirse) | sorghum |
| Triticum aestivum | | Weizen | wheat |
| Xanthium pensylvanicum | Xanthium pens. | Spitzklette | common cocklebur |
| Zea mays | | Mais | Indian corn |

**Tabelle 2:** Selektive Beseitigung von Unkräutern in Erdnüssen und anderen Kulturen bei Nachauflaufanwendung im Gewächshaus

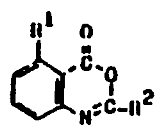

| | | Aufwandmenge | Scädigung (in %) an den Testpflanzen | | | | |
|---|---|---|---|---|---|---|---|
| H¹ | H² | [kg a.S./ha] | Arachis hypogaea | Glycine max | Oryza sativa | Sorghum bicolor | Zea mays |
| Cl | 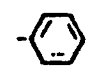 | 1,0 | 0 | 0 | 5 | 0 | 0 |
| H | 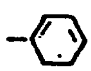 | 1,0 | 0 | 7 | 6 | 0 | 6 |
| | (bekannt aus BE-A-648259) | | | | | | |

0 = keine Schädigung
100 = Pflanzen abgestorben

**Tabelle 3:** Selektive herbizide Wirkung von 4H-3,1-Benzoxazinderivaten bei Nachauflaufanwendung im Gewächshaus

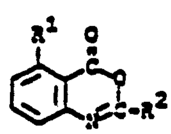

| | | Aufwandmengz | Schädigung (in %) an den Testpflanzen | |
|---|---|---|---|---|
| R¹ | R² | [kg a.S./ha] | Avena sativa | Centaurea spp. |
| ? | 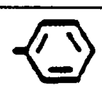 | 3.0 | 0 | 100 |
| Cl | 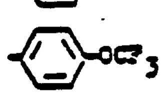 | 3.0 | 0 | 100 |
| Cl | 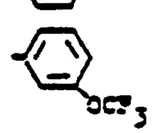 | 3.0 | 0 | 100 |

**Tabelle 4** - Bekämpfung breitblättriger Unkräuter in Cerealien bei Nachauflaufanwendung im Gewächshaus

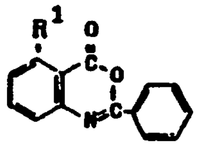

| $R^1$ | Aufwandmenge [kg a.S./ha] | Hordeum vulgare | Oryxa sativa | Triticum sestivum | Chenopod. album | Chrysanth. segetum | Matricaria spp. | Mercurialia annua |
|---|---|---|---|---|---|---|---|---|
| | | Schädigung in % an den Testpflanzen | | | | | | |
| F | 1.0 | 0 | 0 | 0 | 90 | 100 | 99 | 98 |
| Cl | 1.0 | 0 | 6 | 7 | 40 | 50 | 75 | 58 |
| H (bekannt aus BE-A-648259) | 1.0 | 0 | 0 | 0 | 80 | 10 | 0 | 0 |

0 = keine Schädigung
100 = Pflanzen abgestorben

**Tabelle 5** - Selektive Bekämpfung unerwünschter Pflanzen bei Nachauflaufanwendung im Gewächsaus

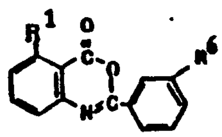

| $R^1R^6$ | Aufwandmenge [kg a.S./ha] | Zea mays | Chenop. album | Desmod. tort. | Euphorb. genic. | Matriha. spp. | Mercurial. annua | Malva neglecta | Solanum nigrum |
|---|---|---|---|---|---|---|---|---|---|
| | | Schädigung in % an den Testpflanzen | | | | | | | |
| F — $OCK_2CK_2H$ | 0.5 | 3 | 100 | 100 | 100 | 100 | 100 | 100 | 100 |
| Cl — $OCF_3$ | 1.0 | 9 | 67 | 92 | 84 | 85 | 45 | 100 | 88 |

0 = keine Schädigung
100 = Pflanzen gestorben

**Tabelle 6:** Selektive Bekämpfung von Unkräutern in Zuckerrüben bei Nachauflaufanwendung am Gewächshaus

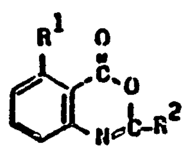

| $R^1$ | $R^2$ | Aufwandmenge [kg a.S./ha] | Beta vulg. | Chenopodium album | Solanum nigrum |
|---|---|---|---|---|---|
| | | | Schädigung in % an den Testpflanzen | | |
| Cl | 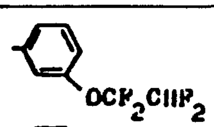 | 1.0 | 3 | 67 | 100 |
| F | 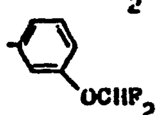 | 1.0 | 0 | 100 | 100 |
| Cl | 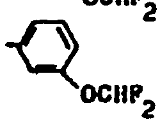 | 1.0 | 0 | 85 | 100 |

0 = keine Schädigung
100 = Planzen gestorben

In Anbetracht der guten Verträglichkeit und der Vielseitigkeit der Applikationsmethoden können die erfindungsgemäßen Herbizide außer bei den in Tabelle 1 aufgeführten Nutzpflanzen noch in einer weiteren großen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

In Betracht kommen beispielsweise die folgenden Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape |
| Brassica napus var. napohrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor — Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfa and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum) Gossypium herbaceum (Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactuca uativa | Kopfsalat | lettuce |
| Lens culinaris | Linse | lentils |
| Linum usitatissimum | Faserlein | flax |

| Botanischer Name | Deutscher Name | Englischer Name |
|---|---|---|
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum app. tuberosum | Wurzelpetersilie | parslay |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsisch | peach trees |
| Pyrus communis | Birne | pear trees |
| Ribes silvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (v. unguiculata) | Kuhbohne | cow peas |
| Vitis vinifera | Weinrebe | grapes |
| Zea mays | Mais | Indian corn, sweet corn, maize |

Zur Verbreiterung des Wirkungsspektrums und zur Erzielung synergistischer Effekte können die 4H-3,1-Benzoxazinderivate der Formel (I) sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, Benzothiadiazinone, 2,6-Dinitroaniline, N-Phenylcarbamate, Thiolcarbamate, Halogencarbonsäuren, Triazine, Amide, Harnstoffe, Diphenyläther, Triazinone, Uracile, Benzofuranderivate und andere in Betracht.

Außerdem ist es nützlich, die erfindungsgemäßen Herbizide, allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phythopathogenen Pilzen bzw. Bakterien. Von Interesse sind ferner die Mischbarkeit mit Mineralstofflösungen, welche zur Behebung von Ernährungs- oder Spurenelementmängeln eingesetzt werden. Es kann auch vorteilhaft sein, die erfindungsgemäßen Mittel einzeln oder in den möglichen Kombinationen zusammen mit festen oder flüssigen Mineraldüngern gemischt auszubringen.

**Patentansprüche** für die Vertragsstaaten BE, CH, DE, FR, GB, IT, LI, LU, NL, SE.

1. 4H-3,1-Benzoxazinderivate der Formel (I)

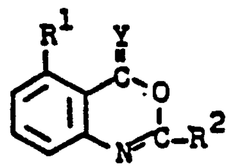

I

in der
Y Sauerstoff oder Schwefel,
$R^1$ Fluor, Chlor oder Brom und
$R^2$ einen durch Chlormethoxy, Fluormethoxy, Difluormethoxy, Difluorchlormethoxy, Trifluormethoxy, Trichlormethoxy, 1,1,2,2-Tetrafluoräthoxy 1,1,2-Trifluor-2-chloräthoxy, 1,1,1-Trifluor-2-bromäthoxy, 1,1,2,3,3,3 Hexafluor-n-propyloxy, Pentafluoräthoxy, Hexafluorisopropoxy, Difluormethylmercapto, Trifluormethylmercapto, Pentafluoräthylmercapto 1,1,2,2-Tetrafluoräthylmercapto, Trichlormethylmercapto oder Dichlorfluormethylmercapto in m- oder p-Stellung substituierten Phenylrest, der auch durch Difluorchlormethylmercapto substituiert sein kann, wenn $R^1$ für Chlor und Y für O steht, oder der auch unsubstituiert sein kann, wenn $R^1$ für Fluor oder Chlor steht, bedeuten.

2. Herbizid, enthaltend ein 4H-3,1-Benzoxazinderivat der Formel (I) gemäß Anspruch 1.

3. Verfahren zur Bekämpfung unerwünschten Pflanzenwuchses, dadurch gekennzeichnet, daß man die Pflanzen oder den Boden mit einem Herbizid, enthaltend ein 4H-3,1-Benzoxazinderivat der Formel (I) gemäß Anspruch 1, behandelt.

4. Verfahren zur Herstellung von 4H-3,1-Benzoxazinderivaten der Formel (I) gemäß Anspruch 1, dadurch gekennzeichnet, daß man eine gegebenenfalls substituierte Anthranilsäure der Formel (II)

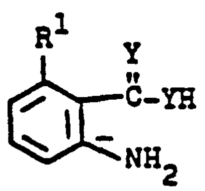

II

in der $R^1$ und Y die im Anspruch 1 genannten Bedeutungen haben,
mit mindestens dem zweifachen molaren Überschuß eines Carbonsäurehalogenids der Formel (III)

$$\text{Hal}-\overset{\text{O}}{\overset{\|}{\text{C}}}-R^2 \qquad \text{III}$$

in der $R^2$ die im Anspruch 1 genannten Bedeutungen hat und Hal für Halogen steht,

in einem aromatischen tertiären Amin als Lösungsmittel bei einer Temperatur im Bereich zwischen 10 und 60°C umsetzt.

5. Verfahren zur Herstellung von 4H-3,1-Benzoxazinderivaten der Formel (I) gemäß Anspruch 1, dadurch gekennzeichnet, daß man eine gegebenenfalls substituierte Anthranilsäure der Formel (II)

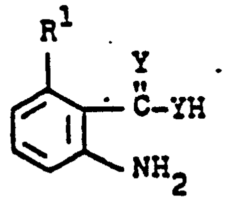

II

in der $R^1$ und Y die im Anspruch 1 genannten Bedeutungen haben,

oder ein Alkalimetall- oder Erdalkalimetallsalz dieser Anthranilsäure mit ungefähr stöchiometrischen Mengen eines Carbonsäurehalogenids der Formel (III)

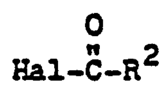

III

in der $R^2$ die im Anspruch 1 genannten Bedeutungen hat und Hal für Halogen steht in einem inerten organischen Lösungsmittel oder in Wasser und gegebenenfalls in Anwesenheit eines Säureacceptors bei einer Temperatur im Bereich von 0 bis 60°C zu einem Carbonamid der Formel (IV)

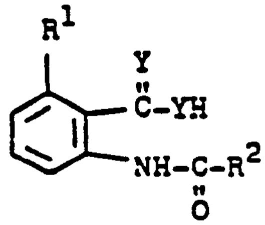

IV

in der $R^1$ $R^2$ und Y die im Anspruch 1 genannten Bedeutungen haben, umsetzt und dieses dann in Gegenwart eines wasserentziehenden Mittels bei einer Temperatur im Bereich zwischen 30 und 150°C cyclisiert.

**Patentansprüche** für den Vertragsstaat AT

1. Herbizid, enthaltend inerte Zusatzstoffe und ein 4H-3,1-Benzoxazinderivat der Formel (I)

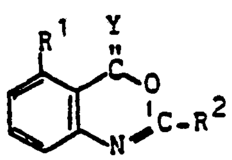

I

in der

Y Sauerstoff oder Schwefel,

$R^1$ Fluor, Chlor oder Brom und

$R^2$ einen durch Chlormethoxy, Fluormethoxy, Difluormethoxy, Difluorchlormethoxy, Trifluormethoxy, Trichlormethoxy, 1,1,2,2-Tetrafluoräthoxy, 1,1,2-Trifluor-2-chloräthoxy, 1,1,1-Trifluor-2-bromäthoxy, 1,1,2,3,3,3-Hexafluor-n-propyloxy, Pentafluoräthoxy, Hexafluorisopropoxy, Difluormethylmercapto, Trifluormethylmercapto, Pentafluoräthylmercapto, 1,1,2,2-Tetrafluoräthylmercapto, Trichlormethylmercapto oder Dichlorfluormethylmercapto in m- oder p-Stellung substituierten Phenylrest, der auch durch Difluorchlormethylmercapto substituiert sein kann, wenn $R^1$ für Chlor und Y für O steht, oder der auch unsubstituiert sein kann, wenn $R^1$ für Fluor oder Chlor steht, bedeuten.

2. Verfahren zur Herstellung von 4H-3,1-Benzoxazinderivaten der Formel (I) gemäß Anspruch 1, dadurch gekennzeichnet, daß man eine gegebenenfalls substituierte Anthranilsäure der Formel (II)

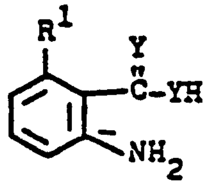

II

in der $R^1$ und Y die im Anspruch 1 genannten Bedeutungen haben, mit mindestens dem zweifachen molaren Überschuß eines Carbonsäurehalogenids der Formel (III)

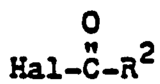

III

in der $R^2$ die in Anspruch 1 genannten Bedeutungen hat und Hal für Halogen steht, in einem aromatischen tertiären Amin als Lösungsmittel bei einer Temperatur im Bereich zwischen 10 und 60°C umsetzt.

3. Verfahren zur Herstellung von 4H-3,1-Benzoxazinderivaten der Formel (I) gemäß Anspruch 1, dadurch gekennzeichnet, daß man eine gegebenenfalls substituierte Anthranilsäure der Formel (II)

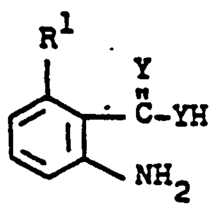

II

in der $R^1$ und Y die im Anspruch 1 genannten Bedeutungen haben, oder ein Alkalimetall- oder Erdalkalimetallsalz dieser Anthranilsäure mit ungefähr stöchiometrischen Mengen eines Carbonsäurehalogenids der Formel (III)

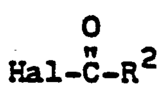

III

in der $R^2$ die is Anspruch 1 genannten Bedeutungen hat und Hal für Halogen steht, in einem inerten organischen Lösungsmittel oder in Wasser und gegebenenfalls in Anwesenheit eines Säureacceptors bei einer Temperatur im Bereich von 0 bis 60°C zu einem Carbonamid der Formel (IV)

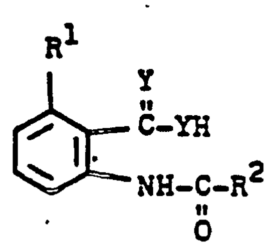

IV

in der $R^1$ $R^2$ und Y die im Anspruch 1 genannten Bedeutungen haben, umsetzt und dieses dann in Gegenwart eines wasserentziehenden Mittels bei einer Temperatur im Bereich zwischen 30 und 150°C cyclisiert.

**Claims** for the Contracting states: BE, CH, DE, FR, GB, IT, LI, LU, NL, SE

1. A 4H-3,1-benzoxazine derivative of the formula (I)

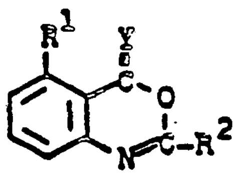

I

where
Y is oxygen or sulfur,
$R^1$ is fluorine, chlorine or bromine, and
$R^2$ is phenyl which is substituted in the m- or p-position by chloromethoxy, fluoromethoxy, difluoromethoxy, difluorochloromethoxy, trifluoromethoxy, trichloromethoxy, 1,1,2,2-tetrafluoroethoxy, 1,1,2-trifluoro-2-chloroethoxy, 1,1,1-trifluoro-2-bromoethoxy, 1,1,2,3,3,3-hexafluoro-n-propyloxy, pentafluoroethoxy, hexafluoroisopropoxy, difluoromethylmercapto, trifluoromethylmercapto, pentafluoroethylmercapto, 1,1,2,2-tetrafluoroethylmercapto, trichloromethylmercapto or dichlorofluoromethylmercapto, and may also be substituted by difluorochloromethylmercapto when $R^1$ is chlorine and Y is oxygen, or may also be unsubstituted when $R^1$ is fluorine or chlorine.

2. A herbicide containing a 4H-3,1-benzoxazine derivative of the formula (I) as claimed in claim 1.

3. A process for combating unwanted plant growth, wherein the plants or the soil are treated with a herbicide containing a 4H-3,1-benzoxazine derivative of the formula (I) as claimed in claim 1.

4. A process for the preparation of a 4H-3,1-benzoxazine derivative of the formula (I) as claimed in claim 1, wherein an unsubstituted or substituted anthranilic acid of the formula (II)

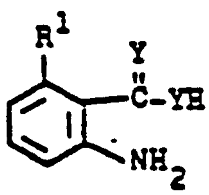

II

where $R^1$ and Y have the meanings given in claim 1, is reacted with at least a twofold molar excess of a carboxylic acid halide of the formula (III)

$$Hal-\overset{O}{\overset{\|}{C}}-R^2$$

III

where $R^2$ has the meanings given in claim 1, and Hal is halogen, in an aromatic tertiary amine as solvent, at from 10 to 60°C.

5. A process for the preparation of a 4H-3,1-benzoxazine derivative of the formula (I) as claimed in claim 1, wherein an unsubstituted or substituted anthranilic acid of the formula (II)

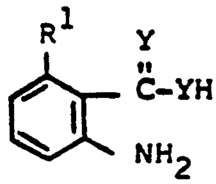

II

where $R^1$ and Y have the meanings given in claim 1, or an alkali metal or alkaline earth metal salt of this anthranilic acid is reacted with about the stoichiometric amount of a carboxylic acid halide of the formula (III)

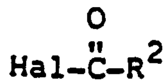

III

where $R^2$ has the meanings given in claim 1, halogen, in an inert organic solvent or in water, in the presence or absence of an acid acceptor, at from 0 to 60° C, to give a carboxylic acid amide of the formula (IV)

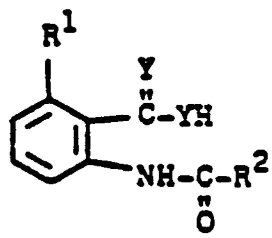

IV

where $R^1$, $R^2$ and Y have the meanings given in claim 1, and this amide is then cyclized at from 30 to 150° C in the presence of a dehydrating agent.

**Claims** for the Contracting state: AT

1. A herbicide containing inert additives and a 4H-3,1-benzoxazine derivative of the formula (I)

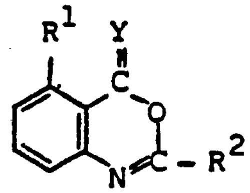

I

where
Y is oxygen or sulfur,
$R^1$ is fluorine chlorine or bromine, and
$R^2$ is phenyl which is substituted in the m- or p-position by chloromethoxy, fluoromethoxy, difluoromethoxy, difluorochloromethoxy, trifluoromethoxy, trichloromethoxy, 1,1,2,2-tetrafluoroethoxy, 1,1,2-trifluoro-2-chloroethoxy, 1,1,1-trifluoro-2-bromoethoxy, 1,1,2,3,3,3-hexafluoro-n-propyloxy, pentafluoroethoxy, hexafluoroisopropoxy, difluoromethylmercapto, trifluoromethylmercapto, pentafluoroethylmercapto, 1,1,2,2-tetrafluoroethylmercapto, trichloromethylmercapto or dichlorofluoromethylmercapto, and may also be substituted by difluorochloromethylmercapto when $R^1$ is chlorine and Y is oxygen, or may also be unsubstituted when $R^1$ is fluorine or chlorine.

2. A process for the preparation of a 4H-3,1-benzoxazine derivative of the formula (I) as defined in claim 1, wherein an unsubstituted or substituted anthranilic acid of the formula (I)

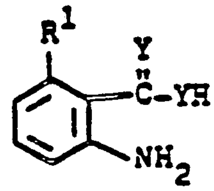

II

where $R^1$ and Y have the meanings given in claim 1, is reacted with at least a twofold molar excess of a carboxylic acid halide of the formula (III)

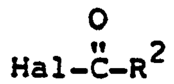 . III

where $R^2$ has the meanings given in claim 1, and Hal is halogen, in an aromatic tertiary amine as solvent, at from 10 to 60°C.

3. A process for the preparation of a 4H-3,1-benzoxazine derivative of the formula (I) as defined in claim 1, wherein an unsubstituted or substituted anthranilic acid of the formula (II)

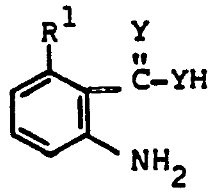 II

where $R^1$ and Y have the meanings given in claim 1, or an alkali metal or alkaline earth metal salt of this anthranilic acid is reacted with about the stoichiometric amount of a carboxylic acid halide of the formula (III)

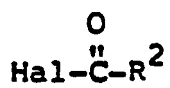 III

where $R^2$ has the meanings given in claim 1, and Hal is halogen, in an inert organic solvent or in water, in the presence or absence of an acid acceptor, at from 0 to 60° C, to give a carboxylic acid amide of the formula (IV)

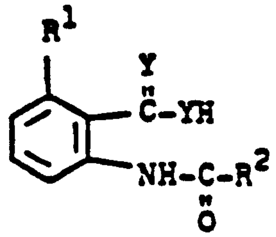 IV

where $R^1$ $R^2$ and Y have the meanings given in claim 1, and this amide is then cyclized at from 30 to 150° C in the presence of a dehydrating agent.

**Revendications** pour les Estats contractants: BE, CH, DE, FR, GB, IT, LI, LU, NL, SE

1. Dérivés de la 4H-3,1-benzoxazine de la formule (I)

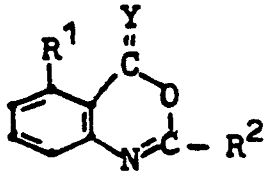 I

dans laquelle
Y désigne un atome d'oxygène ou de soufre,
$R^1$ un atome de fluor, de chlore ou de brome et
$R^2$ un groupe phényle substitué en position méta ou para par un groupe chloro-méthoxy, fluoro-méthoxy, difluoro-méthoxy, difluoro-chlorométhoxy, trifluoro-méthoxy, trichloro-méthoxy, tétrafluor-1,1,2,2 éthoxy, trifluor-1,1,2 chloro-2 éthoxy, trifluor-1,1,1 bromo-2 éthoxy, hexafluor-1,1,2,3,3,3 n-propyl-oxy, pentafluor-éthoxy, hexafluor-isopropoxy, difluorométhyl-mercapto, trifluorométhyl-mercapto, pentafluoréthyl-mercapto,

tétrafluor-1,1,2,2 éthyl-mercapto, trichlorométhyl-mercapto ou dichloro-fluorométhyl-mercapto
ou un groupe phényle substitué par un groupe difluoro-chlorométhyl-mercapto, si $R^1$ = Cl et Y = O
ou un groupe phényle non substitué, si $R^1$ représente un atome de fluor ou de chlore.

2. Composition herbicide, contenant un dérivé de la 4H-3,1-benzoxazine de la formule (I) selon la revendication 1.

3. Procédé de lutte contre le développement indésirable de plantes, caractérisé en ce que l'on traite les plantes ou le sol avec une composition herbicide, contenant un dérivé de la 4H-3,1-benzoxazine de la formule (I) selon la revendication 1.

4. Procédé de préparation de dérivés de la 4H-3,1-benzoxazine de la formule (I) selon la revendication 1, caractérisé en ce que l'on fait réagir un acide anthranilique de la formule (II)

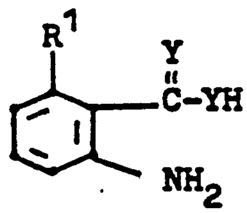

II

dans laquelle $R^1$ et Y possèdent les significations définies dans la revendication 1, et qui peut être substitué,
dans la gamme de températures de 10 à 60°C et dans un solvant choisi parmi les amines aromatiques tertiaires, avec un excès au moins deux fois molaire d'un halogénure d'un acide carboxylique de la formule (III)

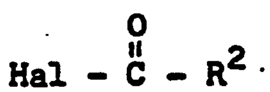

III

dans laquelle $R^2$ possède les significations définies dans la revendication 1 et Hal désigne un atome d'halogène.

5. Procédé de préparation de dérivés de la 4H-3,1-benzoxazine de la formule I selon la revendication 1, caractérisé en ce que l'on fait réagir un acide anthranilique de la formule (II)

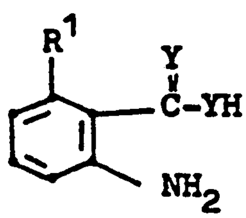

II

dans laquelle $R^1$ et Y possèdent les significations définies dans la revendication 1, et qui peut être substitué,
ou un sel d'un métal alcalin ou d'un métal alcalino-terreux d'un tel acide,
entre 0 et 60°C, dans un solvant organique inerte ou dans de l'eau, le cas échéant en présence d'un fixateur d'acide, avec un halogénure d'acide carboxylique de la formule (III)

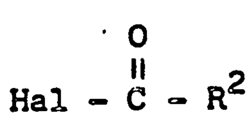

III

dans laquelle $R^2$ possède les significations définies dans la revendication 1 et Hal désigne un atome d'halogène, pour obtenir un amide carboxylique de la formule (IV)

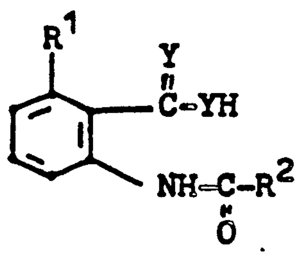

IV

dans laquelle $R^1$, $R^2$ et Y possèdent les significations définies dans la revendication 1, qui est ensuite soumis dans la gamme de températures de 30 à 150°C, en présence d'un agent déshydratant, à une cyclisation.

**Revendications** pour l'Etat contractant: AT

1. Composition herbicide, contenant des additifs inertes et un dérivé de la 4H-3,1-benzoxazine de la formule (I)

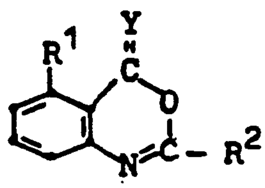

I

dans laquelle
Y désigne un atome d'oxygène ou de soufre,
$R^1$ un atome de fluor, de chlore ou de brome et
$R^2$ un groupe phényle substitué en position méta ou para par un groupe chloro-méthoxy, fluoro-méthoxy, difluoro-méthoxy, difluoro-chlorométhoxy, trifluoro-méthoxy, trichloro-méthoxy, tétrafluor-1,1,2,2 éthoxy, trifluor-1,1,2 chloro-2 éthoxy, trifluor-1,1,1 bromo-2 éthoxy, hexafluor-1,1,2,3,3,3 n-propyloxy, pentafluor-éthoxy, hexafluor-isopropoxy, difluorométhylmercapto, trifluorométhyl-mercapto, pentafluoréthyl-mercapto, tétrafluor-1,1,2,2 éthyl-mercapto, trichlorométhyl-mercapto ou dichloro-fluorométhyl-mercapto
ou un groupe phényle substitué par un groupe difluoro-chlorométhyl-mercapto, si $R^1$ = Cl et Y = O
ou un groupe phényle non substitué, si $R^1$ représente un atome de fluor ou de chlore.

2. Procédé de préparation de dérivés de la 4H-3,1-benzoxazine de la formule (I) selon la revendication 1, caractérisé en ce que l'on fait réagir un acide anthranilique de la formule (II)

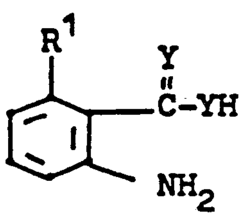

II

dans laquelle $R^1$ et Y possèdent les significations définies dans la revendication 1, et qui peut être substitué, dans la gamme de températures de 10 à 60°C et dans un solvant choisi parmi les amines aromatiques tertiaires, avec un excès au moins deux fois molaire d'un halogénure d'un acide carboxylique de la formule (III)

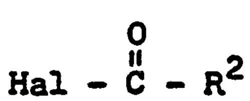

III

dans laquelle $R^2$ possède les significations définies dans la revendication 1 et Hal désigne un atome d'halogène.

3. Procédé de préparation de dérivés de la 4H-3,1-benzoxazine de la formule (I) selon la revendication 1, caractérisé en ce que l'on fait réagir un acide anthranilique de la formule (II)

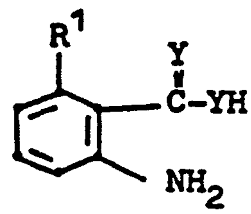

II

dans laquelle $R^1$ et Y possèdent les significations définies dans la revendication 1, et qui peut être substitué, ou un sel d'un métal alcalin ou d'un métal alcalino-terreux d'un tel acide,

entre 0 et 60° C, dans un solvant organique inerte ou dans de l'eau, le cas échéant en présence d'un fixateur d'acide, avec un halogénure d'acide carboxylique de la formule (III)

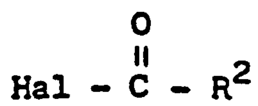

III

dans laquelle $R^2$ possède les significations définies dans la revendication 1 et Hal désigne un atome d'halogène, pour obtenir un amide carboxylique de la formule (IV)

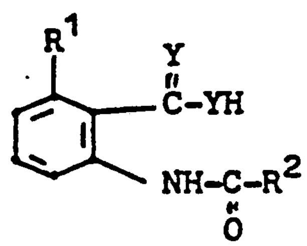

IV

dans laquelle $R^1$, $R^2$ et Y possèdent les significations définies dans la revendication 1, qui est ensuite soumis dans la gamme de températures de 30 à 150° C, en présence d'un agent déshydratant, à une cyclisation.